# EUROPEAN PATENT SPECIFICATION

(11) **EP 1 689 381 B1**
(45) Date of publication and mention of the grant of the patent: **24.02.2010**
(21) Application number: 04764249.1
(22) Date of filing: 18.08.2004
(51) Int. Cl.: A61K 31/198, A61K 31/401, A61K 31/375, A61K 31/353, A61K 36/82, A61K 33/06, A61K 33/04, A61K 33/34, A61K 33/32, A61P 9/00, A61P 9/10

(54) **PHARMACEUTICAL COMPOSITION COMPRISING I.A. VITAMIN C, MAGNESIUM, GREEN TEA EXTRACT FOR RETARDING CARDIOVASCULAR DISEASES (SMC PROLIFERATION)**
PHARMAZEUTISCHE ZUSAMMENSETZUNG UMFASSEND U.A. VITAMIN C, MAGNESIUM, EXTRAKT AUS GRÜNEM TEE ZUR VERZÖGERUNG VON KARDIOVASKULÄREN ERKRANKUNGEN (SMC PROLIFERATION)
COMPOSITION PHARMACEUTIQUE, COMPRENANT NOTAMMENT DE LA VITAMINE C, DU MAGNESIUM ET DE L'EXTRAIT DE THE VERT, DESTINEE A RETARDER DES MALADIES CARDIOVASCULAIRES (PROLIFERATION DES SMC)

(30) Priority: 05.09.2003 US 657019; 15.03.2004 US 801262
(43) Date of publication of application: 16.08.2006
(73) Proprietor: Rath, Matthias, 6422 PC Heerlen (NL)
(72) Inventor: RATH, Matthias, 6422 PC Heerlen (NL); NIEDZWIECKI, Aleksandra, San Jose, CA 95129 (US); IVANOV, Vadim, Castro Valley, CA 94552 (US); ROOMI, Waheed, Sunnyvale, CA 94086 (US)
(74) Representative: Sacht-Gorny, Gudrun
(86) International application number: PCT/EP2004/009263
(87) International publication number: WO 2005/023239

(56) References cited:
- WO-A-01/13901
- WO-A-03/057201
- US-A1- 2003 170 319
- IVANOV V.: "EPICAN FORTE, a mixture of ascorbic acid, lysine, proline, arginine, cysteine and green tea extract suppresses autocrine inflammatory respone in cultured human aortic smooth muscle cell" 14 July 2003 (2003-07-14), pages 1-6, XP002305410 Retrieved from the Internet: URL:http://www.cardiologyonline.com/ichd03 /advance_prog_mon_p.htm> [retrieved on 2004-11-12]
- DATABASE WPI Section Ch, Week 198321 Derwent Publications Ltd., London, GB; Class B04, AN 1983-49916K XP002308013 & JP 58 062117 A (YOSHIHIRO F) 13 April 1983 (1983-04-13)
- AIZIKOV M I ET AL: "EFFECT OF POLYPHENOLS ON LIPID METABOLISM IN HYPERLIPEMIA AND ATHEROSCLEROSIS" CHEMICAL ABSTRACTS + INDEXES, AMERICAN CHEMICAL SOCIETY. COLUMBUS, US, vol. 106, 1987, page 39, XP002943855 ISSN: 0009-2258
- JANE HIGDON, LINUS PAULING INSTITUTE: "TEA" INTERNET ARTICLE, 7 December 2002 (2002-12-07), pages 1-7, XP002305232 [retrieved on 2004-11-11]

## Description

The present invention provides pharmaceutical compositions for alleviating or retarding cardiovascular diseases which are characterized by excessive smooth muscle cell proliferation in a mammal, comprising lysine, proline, arginine, vitamin C, magnesium, green tea extract, N-acetyl-cysteine, selenium, copper, manganese and one pharmaceutical acceptable component selected from the group consisting of a carrier, a diluent, and an excipient, wherein the pharmaceutical composition without the acceptable component contains 7-9 wt % magnesium, 20-30 wt % ascorbic acid and 11-25 wt % green tea extract.

Cardiovascular disease is the major cause of mortality in western countries and around the world. The majority of adverse cardiovascular events are caused by the development of atherosclerotic lesions in coronary and cerebral arteries. Abnormal growth of arterial smooth muscle cells is one of the most important steps in the development of atherosclerosis. In response to pathological stimuli, smooth muscle cells first migrate from the media layer to the intima layer of the arterial wall, and then proliferate within the intima layer. These events are crucial in the initial development of atherosclerotic plaques. Formation of atherosclerotic lesions in the intima layer occurs in many cardiovascular diseases including hypertension, atherosclerosis, myocardial ischemia, infarction and stroke. (R. Ross, Cellular Mechanisms of Atherosclerosis, Atherosclerosis Review, 103, Vol. 25, pages 195-200). Therefore, it is desirable to prevent pathological stimulation of smooth muscle growth. Inflammatory response, either acute or chronic low level, is an aggravating factor which stimulates and accelerates the development of atherosclerotic lesions. As a part of the atherosclerotic process there is monocyte infiltration of blood vessel walls, including the endothelium. The monocytes that remain inside the arterial wall accumulate oxidized lipids and become overloaded with lipids, and become foam cells. Foam cells are one of the major characteristics of atherosclerotic lesions. Thus, it is desirable to decrease or retard the formation of these foam cells in the arterial wall and recruitment of monocytes from the blood stream to the arterial walls. Monocytes are attracted to the arterial wall in response to secretion of inflammatory mediators by arterial smooth muscle cells. These mediators include MCP-1. Expression of adhesive molecules such as P-Selectin and ICAM -1 facilitates the process of monocyte's initial adhesion to the arterial wall and consequent penetration inside the arterial wall. The inflammatory response cascade also includes Interleukin 6 (IL-6) and Interleukin 1 (IL-1) expression and secretion. These mediator are responsible for at least two functions. They trigger systemic inflammatory response including further monocyte recruitment. The other effect is that cytokines can effect smooth muscle cells in autocrine reactions, which means that cells continue to release these inflammatory mediators in a vicious cycle of pathological cell stimulation. Therefore, it is also desirable to reduce the expression and release of inflammatory response mediators such as IL-1 , IL-6, MCp-1, and others.
There is a long felt need to provide a safe and effective pharmaceutical composition and method for alleviating pathological compositions in mammals, primarily those of cardiovascular abnormalities, and those associated with chronic or low level inflammatory response. There is also a need for compounds and substances for the treatment of atherosclerosis and inflammation, that do not have side effects. There is yet another need for compounds and substances in the retardation of development of atherosclerosis, arteriosclerosis, and retardation of chronic and low level inflammatory response using low cost non-drug substances and compounds instead of expensive drugs.

The international application WO 03/057201 discloses a nutrient pharmaceutical composition comprising an ascorbic compound, a lysine compound, a proline compound and a polyphenol compound useful in treating cancer and also inflammatory diseases. Ivanov et al. discloses in a poster session of an International Congress in Washington DC, USA on July 14, 2003 a mixture of ascorbic acid, lysine, proline, arginine, cysteine and green tea extract suppressing autocrine inflammatory response in smooth muscle cells.

Accordingly, the technical problem underlying the present invention could be seen as the provision of means and methods complying with the aforementioned needs. Specifically, it is an object of the present invention to provide pharmaceutical compositions for alleviating or retarding cardiovascular diseases which are characterized by excessive smooth muscle cell proliferation in a mammal, comprising lysine, proline, arginine, vitamin C, magnesium, green tea extract, N-acetyl-cysteine, selenium, copper, manganese and one pharmaceutical acceptable component selected from the group consisting of a carrier, a diluent, and an excipient, wherein the pharmaceutical composition without the acceptable component contains 7-9 wt % magnesium, 20-30 wt % ascorbic acid and 11-25 wt % green tea extract. The cardiovascular diseases include atherosclerosis and arteriosclerosis.
The technical problem is solved, i.e. the cardiovascular diseases are treated, by the embodiments characterized in the claims and hereinafter. The scope of the invention is defined by the appended claims. Thus, the present invention provides pharmaceutical compositions and methods for the treatment of cardiovascular diseases which are characterized by excessive smooth muscle cell proliferation.

Accordingly, the present invention provides pharmaceutical compositions for alleviating or retarding cardiovascular diseases which are characterized by excessive smooth muscle cell proliferation in a mammal, comprising lysine, proline, arginine, vitamin C, magnesium, green tea extract, N-acetyl-cysteine, selenium, copper, manganese and one pharmaceutical acceptable component selected from the group consisting of a carrier, a diluent, and an excipient, wherein the pharmaceutical composition without the acceptable component contains 7-9 wt % magnesium, 20-30 wt % ascorbic acid and 11-25 wt % green tea extract.

Preferably, the mammal referred to in accordance with the present invention is a human, dog, cat, or horse. More preferably, the human is a post-menopausal woman.

The present invention provides a method for alleviating pathological conditions related to atherosclerosis, arteriosclerosis, in a mammal comprising the step of administering to the mammal in need of treatment an effective amount of the pharmaceutical composition comprising the further compounds in the following ratios: approximately 25,000 parts of lysine, approximately 15,000 parts of proline, approximately 8,000 parts of arginine, approximately 80,000 parts of ascorbic acid, approximately 30,000 parts of magnesium, approximately 50,000 parts of green tea extract, approximately 15,000 parts of N-acetyl-cysteine, approximately 5 parts of selenium, approximately 50 parts of copper, and approximately 200 parts of manganese wherein the pharmaceutical composition contains 7-9 wt % magnesium, 20-30 wt % ascorbic acid and 11-25 wt % green tea extract. It is to be understood that the parts referred to in accordance with the invention are not absolute values. Rather, they are used to define the ratios in which the substances shall be present. For example, if a composition comprises 12.5 parts of lysine and 7.5 parts of proline, the composition will contain lysine and proline in a ratio of 25,000 parts lysine (12,5 times 2,000) and 15,000 parts proline (7.5 times 2,000) as referred to in accordance with the present invention. More preferably, the pharmaceutical composition to be administered as specified above comprises approximately 25 mg of lysine, approximately 15 mg of proline, approximately 8 mg of arginine, approximately 80 mg of ascorbic acid, approximately 30 mg of magnesium, approximately 50 mg of green tea extract, approximately 15 mg of N-acetyl-cysteine, approximately 5 mcg of selenium, approximately 50mcg of copper, and approximately 200 mcg of manganese wherein the pharmaceutical composition contains 7-9 wt % magnesium, 20-30 wt % ascorbic acid and 11-25 wt % green tea extract.

The present invention provides a method for alleviating pathological conditions related to atherosclerosis, arteriosclerosis, in a mammal comprising the step of administering to the mammal in need of treatment an effective amount of the pharmaceutical composition of lysine, proline, arginine, vitamin C, magnesium, green tea extract, N-acetyl-cysteine, selenium, copper, manganese and one pharmaceutical acceptable component selected from the group consisting of a carrier, a diluent, and an excipient, wherein the pharmaceutical composition contains 7-9 wt % magnesium, 20-30 wt % ascorbic acid and 11-25 wt % green tea extract.

Optionally, the effective amount of the composition is a daily dosage of approximately 0.3 mg/kg lysine, 0.2 mg/kg proline, 0.1 mg/kg arginine, 1.1 mg/kg Vitamin C, 0.4 mg/kg magnesium, 0.7 mg/kg green tea extract, and 0.2 mg/kg N-acetyl-cysteine.

Preferably, the pharmaceutical composition is in oral or parenteral form. More preferably, the oral form is a tablet or a capsule. Preferably, the pharmaceutical compositions may be administered orally, intravenously, or parenterally.

The present invention provides compositions for treating pathological conditions associated with chronic or low level inflammatory response, comprising lysine, proline, arginine, ascorbic acid, magnesium, green tea extract, N-acetyl-cysteine, selenium, copper, and manganese.

Through administration of a single pill or capsule, the composition delivers a dosage of approximately 0.3 mg/kg lysine, 0.2 mg/kg proline, 0.1 mg/kg arginine, 1.1 mg/kg Vitamin C, 0.4 mg/kg magnesium, 0.7 mg/kg green tea extract, and 0.2 mg/kg N-acetyl-cysteine. The administration of the pill or capsule is optionally repeated per day to be effective in retarding chronic or low level inflammation.

The pharmaceutical compositions of the present invention have shown to be effective in inhibiting smooth cell proliferation. The compositions have therefore clinical relevance in applications such as antihypertensive agents. By reducing smooth muscle cell proliferation, the compositions act as a vasodilator and decreases total peripheral vascular resistance. Moreover, the pharmaceutical compositions of the present invention are shown to inhibit the smooth muscle proliferation which is at cause of development and progression of atherosclerosis. In vitro experiments underlying the present invention show the potent effects of the compositions as inhibitors of proliferation (measured by ³H-thymidine incorporation). Thereby, the compositions will attenuate the onset of atherosclerosis. The pharmaceutical compositions of the present invention also inhibit smooth muscle migration and thus attenuate the development and progression of atherosclerosis. Chemotactic migration of medial smooth muscle cells into the intima is an important first step in the pathogenesis of neo-intima formation during atherosclerosis. PDGF is believed to be a key substance for promoting smooth muscle cell migration. (Russel R. (1986) N. Engl. J. Med. 314 488-500). The ability of the compositions disclosed herewith to inhibit myo-intimal formation in vivo may in part be related to direct inhibition of the physical migration of vascular smooth muscle from the tunic a media into the tunica intima. Accordingly, in another embodiment, the present invention provides pharmaceutical compositions comprising lysine, proline, arginine, ascorbic acid, magnesium, green tea extract, N-acetyl-cysteine, selenium, copper, and manganese, and a pharmaceutically acceptable excipient, for inhibiting proliferation of smooth muscle cells in mammals, preferably human beings, particularly for inhibiting proliferation in blood vessels of those in risk of development of heart disease; for inhibiting the development of atherosclerosis. In another embodiment, the present invention also provides a method of treatment for inhibition of proliferation and migration of smooth muscle cells in mammals, preferably human beings, particularly a method of treatment for preventing proliferation in blood vessels of those in risk of development of heart disease, for inhibiting the development of atherosclerosis; said method comprising administering to a patient in need thereof an effective dose of a pharmaceutical composition disclosed herein comprising lysine, proline, arginine, ascorbic acid, magnesium, green tea extract, N-acetyl-cysteine, selenium, copper, and manganese, and a pharmaceutically acceptable excipient thereof. Compositions of the present invention are shown to be effective in inhibiting vascular smooth muscle cell proliferation and migration mediated by a wide variety of different mitogens.
The compositions of the present invention are effective in inhibiting estrogen-induced smooth muscle cell proliferation and invasion. They will regulate the blood pressure as well as development of atheroscloerotic plaques. The combined effect of ingredients such as lysine and proline may prevent severe connective tissue degradation which in turn may attenuate the process of proliferation and invasion. Additionally, green tea extract and vitamin C may also blunt the connective tissue degradation by virtue of their anti-oxidant property. It has been surprisingly found that the ingredients present in the compositions of the invention act synergistically in counteracting the estrogen's effects of cardiovascular degradation and cancer development. Advantageously, there is provided a safe and effective pharmaceutical composition and therapeutic method for alleviating the aforementioned pathological compositions in mammals having no detectable side effects associated with the compounds and substances of the invention. Furthermore, the pharmaceutical compositions and therapeutic methods and uses of the invention using low cost non-drug substances and compounds instead of expensive drugs.

As used herein, the term "alleviating" is used to mean reducing, inhibiting, attenuating or treating the syndromes accompanied with the pathological conditions referred to in accordance with the present invention. Alleviation becomes apparent for the clinician by monitoring the symptoms accompanied with the said pathological conditions. The symptoms are described in detail in standard text books such as Stedman or Pschyrembel. Alleviation preferably refers to significant reduction, inhibition, attenuation or treatment. The significance can be determined by standard methods of statistics, e.g., Student's t-test, chi square test and others. Preferably, the syndromes referred to in accordance with the present invention are those common to post-menopausal women receiving estrogen therapy. "Syndromes of estrogen therapy" is a well-recognized term and refers predominately herein to cardiovascular and neoplastic problems in women receiving estrogen replacement therapy including hypertension, atheroscloerosis and breast cancer.

The term "effective amount" means an amount of composition of the present invention which is capable of alleviating the symptoms of the various pathological conditions herein described.

The term "pharmaceutically acceptable" in reference to carriers, diluents, and excipients means that they must be compatible with the other ingredients of the formulation, and not deleterious to the recipient thereof.

"Wt %" refers to % of the ingredient as a proportion of the total weight of the composition; for example, 25 wt % of lysine indicates that 25 % of the total weight of the composition is made up of lysine.

The term "approximately" means that the value referred to may vary to a certain degree. The value, however, must be sufficient to achieve the effects of the compositions referred to in accordance with the present invention. Whether a given value of a compound achieves these effects can be determined by standard techniques including those assays referred to in the accompanied Example. Preferably, the variation of the values lie within the standard deviation as calculated by the statistical tests referred to in accordance with the present invention. More preferably, the variation of the values is ± 20%,± 15%, ± 10%, ± 5%, ± 2%, ± 1% or ± 0.5%. Most preferably, the term "approximately" refers to the specific values.

"Lysine" as used herein comprises lysine salts, preferably hydroxylysine and hydroxylysine salts. Typically, the L-lysine is administered in a daily dose of approximately 0.3 mg/kg. L-lysine may be administered orally in a dosage form once, twice or three times a day. For an average individual weighing 72 kg, the recommended total amount of lysine per single administration is approximately 25 milligrams (mg).

"Proline" as used herein comprises proline, proline salts, hydroxyproline and hydroxyproline salts. Typically, the L-proline is administered in a daily dose of approximately 0.2 mg/kg. L-proline may be administered orally in a dosage form once, twice or three times a day. For an average individual weighing 72 kg, the recommended total amount of proline per single administration is approximately 15 milligrams (mg).

"Arginine" as used herein comprises arginine and arginine salts. Typically, the L-arginine is administered in a daily dose of approximately 0.1 mg/kg. L-arginine may be administered orally in a dosage form once, twice or three times a day. For an average individual weighing 72 kg, the recommended total amount of arginine per single administration is approximately 8 mg.

"Vitamin C" as used herein comprises ascorbic acid, ascorbate salts and its derivatives. As used herein, ascorbic acid and vitamin C are used interchangeably and, preferably, include calcium ascorbate, magnesium ascorbate or ascorbyl palmitate. Typically, ascorbic acid is administered in a daily dose of approximately 0.4 mg/kg. Ascorbic acid may be administered orally in a dosage form once, twice or three times a day. For an average individual weighing 72 kg, the recommended total amount of ascorbic acid per single administration is approximately 80 mg. The different compounds claimed in this application can be used together in the form of covalently bound compounds or as physical mixture or in any other combination.

The present invention provides pharmaceutical compositions comprising an ascorbate compound, proline, lysine, or any combination thereof. Therefore, the present invention is not limited to ascorbate, proline or lysine, but embodies any equivalent structures that may be used in accordance with the preferred uses of the present invention.

"Green tea extract" as used herein refers to polyphenolic compounds that are present in green tea. Polyphenolic compounds may be present as up to 30% dry weight in green tea. They include flavanols, flavandiols, flavonoids, and phenolic acids. Flavanols represent the most abundant polyphenols in green tea and are commonly known as catechins. The major catechins in green tea extract include: 1) (-)-epicatechin, 2) (-)-epicatechin-3-gallate, 3) (-)-epigallocatechin, and 4) (-)-epigallocatechin-3-gallate (EGCG). Among the catechins, EGCG is the major polyphenolic constitutent present in green tea. As used herein, green tea extract contains about 80% by weight polyphenols and is free of caffeine.

Green tea extract may be administered in a daily dose of approximately 0.7 mg/kg. Green tea extract may be administered orally in a dosage form once, twice or three times a day. For an average individual weighing 72 kg, the recommended total amount of green tea extract per daily administration is approximately 50 mg.

"N-acetyl-cysteine" as used herein comprises cysteine or cystine (dimer of cysteine) and cysteine salts thereof. N-acetyl-cysteine may be administered in a daily dose of approximately 0.2 mg/kg. N-acetyl-cysteine may be administered orally in a dosage form once, twice or three times a day. For an average individual weighing 72 kg, the recommended total amount of N-acetyl-cysteine per single administration is approximately 15 mg.

The present invention further provides minerals and/or trace element. Trace elements may help to catalyze the production of these macromolecules needed for connective tissues. Preferred trace elements in accordance with the present invention are iron, copper, zinc, manganese, cobalt, molybdenum, selenium, chromium, nickel, tin, fluorine or vanadium.

Magnesium may be administered in a daily dose of approximately 0.7 mg/kg. Magnesium may be administered orally in a dosage form once, twice or three times a day. For an average individual weighing 72 kg, the recommended total amounts of magnesium per single administration is approximately 30 mg.

Selenium may be administered in a daily dose of approximately 0.00007 mg/kg. Selenium may be administered orally in a dosage form once, twice or three times a day. For an average individual weighing 72 kg, the recommended total amount of selenium per single administration is 5 mcg.

Copper may be administered in a daily dose of approximately 0.0007 mg/kg. Copper may be administered orally in a dosage form once, twice or three times a day. For an average individual weighing 72 kg, the recommended total amount of copper per single administration is approximately 50 mcg.

Manganese may be administered in a daily dose of approximately 0.04 mg/kg. Manganese may be administered orally in a dosage form once, twice or three times a day. For an average individual weighing 72 kg, the recommended total amount of manganese per single administration is approximately 200 mcg.

According to the present invention, some ingredients of the composition are present at a high amount. Vitamin C is present between 20-30 wt % in comparison to the total composition (compared to the total composition which does not include the carrier, excipient, fillers, additives etc.). Green tea extract is present between 11-25 wt % (compared to the total composition), and magnesium is present between 7-9 % (compared to the total composition).

The composition optionally includes one or more of the following substances; Vitamin A, Vitamin D3, Vitamin E, Vitamin B1, Vitamin B2, Niacin, Vitamin B6, Folic Acid Vitamin B12, Biotin, Pantothenic Acid, Calcium, Phosphorus, Zinc, Chromium, Molybdenum, Pottasium, Citrus Bioflavonoids, Inositol, L-Carnitine, CoEnzyme Q10, Glucosamine, Taurine, and Chondroitin Sulfate. The substances are preferably comprised by said composition in the following ratios: 191 IU (International unit) of Vitamin A, 20 IU of Vitamin D3, 10 IU of Vitamin E, 1,500 parts of Vitamin B1, 1,500 parts of mg of Vitamin B2, 10,000 parts of Niacin, 1,500 parts of Vitamin B6, 75 parts of folic acid, 3.3 parts of Vitamin B12, 10 parts of Biotin, 5,000 parts of Pantothenic Acid, 15,000 parts of Calcium, 2,500 parts mg of Phosphorus, 2,500 parts of Zinc, 5 parts of Chromium, 0.5 parts of Molybdenum, 5,000 parts of Pottasium, 15,000 parts of Citrus Bioflavonoids, 5,000 parts of Inositol, 5,000 parts of L-Carnitine, 2,500 parts of CoEnzyme Q10, 25,000 parts of Glucosamine (N-Acetyl-D-Glucosamine), 50,000 parts of Taurine, and 15,000 parts of Chondroitin Sulfate. More preferably, the amounts of these substances are optionally approximately as follows; 191 IU (International Units) of Vitamin A, 20 IU of Vitamin D3, 10 IU of Vitamin E, 1.5 mg of Vitamin B1, 1.5 mg of Vitamin B2, 10 mg of Niacin, 1.5 mg of Vitamin B6, 75 mcg of folic acid, 3.3 mcg of Vitamin B12, 10 mcg of Biotin, 5 mg of Pantothenic Acid, 15 mg of Calcium, 2.5 mg of Phosphorus, 2.5 mg of Zinc, 5 mcg of Chromium, 0.5 mcg of Molybdenum, 5 mg of Pottasium, 15 mg of Citrus Bioflavonoids, 5 mg of Inositol, 5 mg of L-Carnitine, 2.5 mg of CoEnzyme Q10, 25 mg of Glucosamine (N-Acetyl-D-Glucosamine), 50 mg of Taurine, and 15 mg of Chondroitin Sulfate.

The compositions of the present invention are useful in treating or inhibiting cardiovascular diseases which are characterized by excessive smooth muscle cell proliferation (smooth muscle cell hyperproliferation). Whether a disease is characterized by excessive smooth muscle cell proliferation can be determined by the clinician using standard techniques. Such standard techniques may comprise antibody based in vivo techniques, biopsy followed by histological tissue sample analysis or flow cytometry of tissue samples. The compositions are particularly useful in treating hypertension and atherosclerosis which frequently arise due to smooth muscle cell hyperproliferation. Atherosclerosis is associated with cholesterol metabolism. It is known that a weakened connective tissue promotes plaque formation in arterial walls. Thus, the present invention provides an ascorbate compound, lysine and proline in an effective amount to strengthen the connective tissue. Ascorbate is known to stimulate the synthesis of collagen, elastin and other connective tissue macromolecules from fibroblast and related cells. The amino acids lysine and proline are the predominant amino acids required for the synthesis of connective tissue molecules.

The dosage requirements vary with the route of administration, the severity of the symptoms presented and the particular subject being treated. A recommended daily dosage of the composition would be administered orally. It is recommended for a daily dosage of approximately 0.3 mg/kg lysine, 0.2 mg/kg proline, 0.1 mg/kg arginine, 1.1 mg/kg Vitamin C, 0.4 mg/kg magnesium, 0.7 mg/kg green tea extract, and 0.2 mg/kg N-acetyl-cysteine. The administration of the pill or capsule is optionally repeated per day to be effective in retarding chronic or low level inflammation.

The compositions of the present invention may be administered by a variety of routes which include, but are not limited to oral, intravenous, or parenteral administration. Precise dosages for oral, intravenous, or parenteral administration may vary and will be determined based on experience with the individual subject treated. Preferably, the pharmaceutical composition is in unit dosage form, e.g. as tablets or capsules. In such form, the composition is sub-divided into unit doses containing appropriate quantities of the active ingredient; the unit dosage forms can be packaged compositions, for example, packaged powders, vials, or ampoules. The unit dosage form can be, for example, a capsule, a pill or tablet itself, or it can be the appropriate number of any such compositions in package form.

Pharmaceutical formulations of the present invention can be prepared by procedures known in the art using well known and readily available ingredients. For example, the ingredients of the present compositions can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such was carboxymethyl cellulose and other cellulose derivatives, alginates, gelatin, and polyvinyl-pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, and glycerol monostearate; adsorptive carriers such as kaolin and bentonite; and lubricants such as talc, calcium and magnesium stearate, and solid polyethyl glycols.

The therapeutic compounds of the present invention may be formulated into pharmaceutical compositions that may optimize or facilitate their use. In particular, the pharmaceutical compositions contains effective amounts for the treatment of athersclerosis, arteriosclerosis, and retardation of chronic or low level inflammation response. Such pharmaceutical compositions often contain a pharmaceutically acceptable carrier or diluent, and if appropriate, an excipient.

The compositions also can be formulated as elixirs or solutions for convenient oral administration or as solutions appropriate for parenteral administration, for example, by intramuscular, subcutaneous or intravenous routes. Ideally the formulation is in the form of a pill, tablet, capsule, lozenge, liquid or similar dosage form. The compositions may well be suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Pharmaceutical formulations of the present invention can be prepared by procedures known in the art using well known and readily available ingredients. For example, the compounds of formula I, with or without an estrogen or progestin compound, can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl-pyrrolidone; moisturizing agents such as glycerol; disintegrating agents such as calcium carbonate and sodium bicarbonate; agents for retarding dissolutions such as paraffin; resorption accelerators such as quaternary ammonium compounds; surface active agents such as cetyl alcohol, and glycerol monostearate; adsorptive carriers such as kaolin and bentonite; and lubricants such as talc, calcium and magnesium stearate, and solid polyethyl glycols.

The compounds also can be formulated as elixirs or solutions for convenient oral administration or as solutions appropriate for parenteral administration, for example, by intramuscular, subcutaneous or intravenous routes. Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Unless otherwise defined, all scientific terms used herein have the same meaning as commonly understood by one of ordinary skill in the art. Exemplary methods and materials are described below and their equivalents can be used.

The figures show:

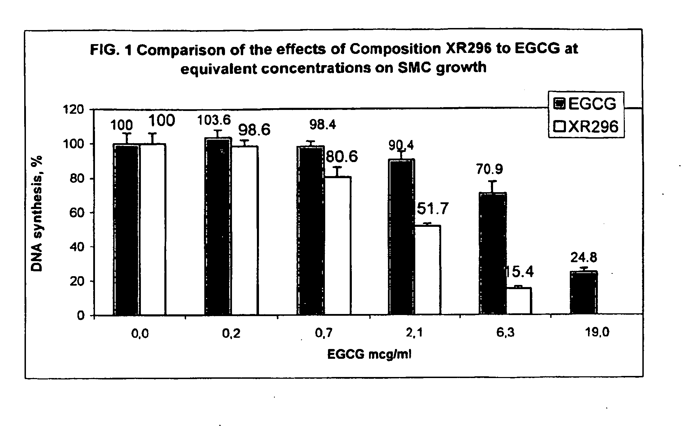

**FIG. 1** shows the effect of XR296 on inhibition of smooth muscle cell growth in comparison to EGCG at equivalent concentrations. [³H] thymidine incorporation in human smooth muscle cells is an indicator of de novo DNA synthesis and cell growth. Thymidine incorporation is expressed as a percentage of the value for the control group (100%).

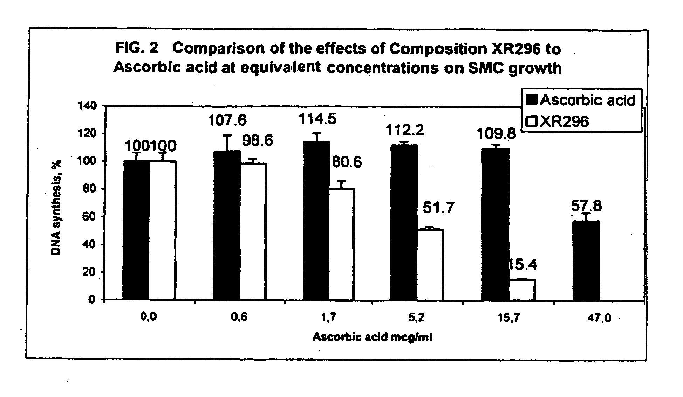

**FIG. 2** shows the effect of XR296 on inhibition of smooth muscle cell growth in comparison to Ascorbic Acid at equivalent concentrations. [³H] thymidine incorporation in human smooth muscle cells is an indicator of de novo DNA synthesis and cell growth. Thymidine incorporation is expressed as a percentage of the value for the control group (100%).

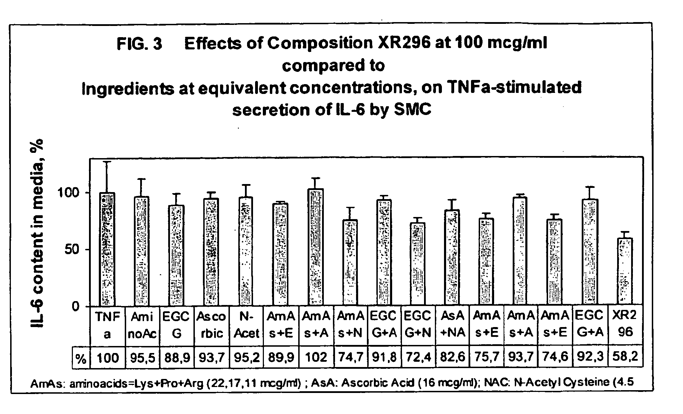

**FIG. 3** is a graph that compares the effect of the composition (XR296)(at 100 mcg/ml) of the present invention to the effect of some of its individual components in concentrations present in XR296, on TNFα stimulated secretion of IL-6 in smooth muscle cells.

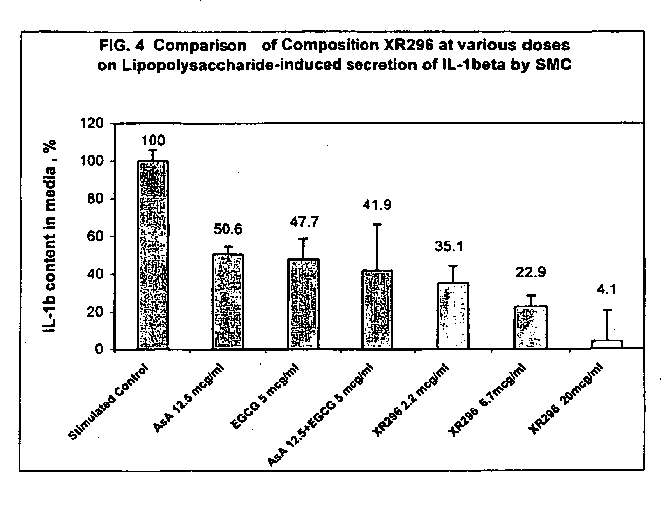

**FIG. 4** is a graph that compares the effect of the composition (XR296)(at 20 mcg/ml) of the present invention to the effect of some of its individual components in concentrations equivalent to four times (80 mcg/ml) present in XR296, on lipopolysaccharide-induced secretion of IL-1β by smooth muscle cells.

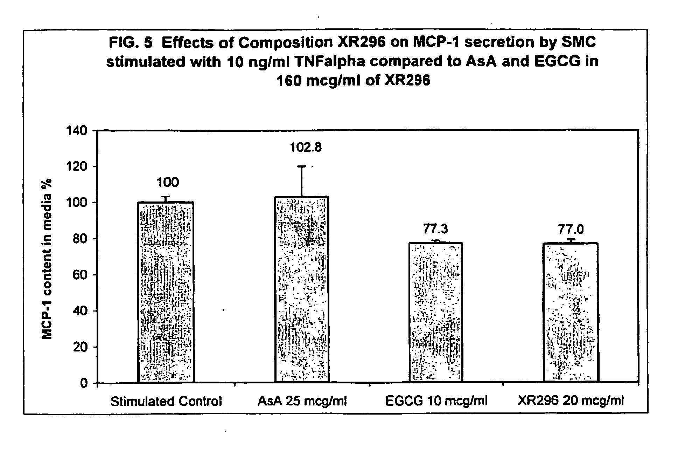

**FIG. 5** is a graph that compares the effect of the composition (XR296)(at 20 mcg/ml) of the present invention to the effect of some of its individual components in concentrations equivalent to 160 mcg/ml of XR296, on 10. ng/ml TNFα stimulated secretion of MCP-1 by smooth muscle cells.

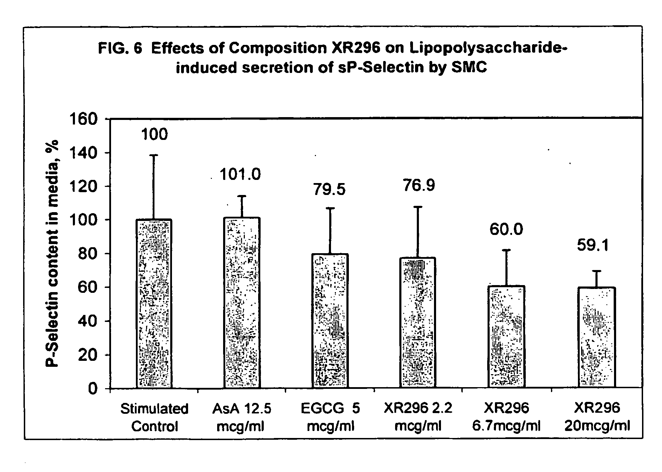

**FIG. 6** is a graph that compares the effect of the composition (XR296)(at 2.2 mcg/ml, 6.7 mcg/ml, and 20 mcg/ml) of the present invention to the effect of some of its individual components in concentrations equivalent to four times (80 mcg/ml) present in XR296, on lipopolysaccharide-induced secretion of P-Selectin by smooth muscle cells.

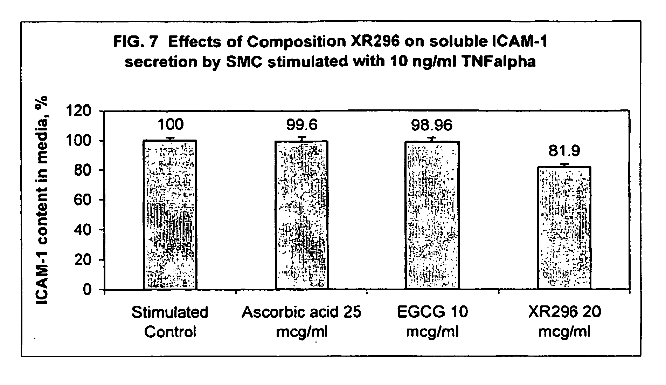

**FIG. 7** is a graph that compares the effect of the composition (XR296)(at 20 mcg/ml) of the present invention to the effect of some of its individual components in concentrations equivalent to 160 mcg/ml of XR296, on 10 ng/ml TNFα stimulated secretion of ICAM-1 by smooth muscle cells.

### Example: Experimental Rationale and Protocols

### Growth Rate Assay for Smooth Muscle Cells

Rationale: As described, the excessive growth rate of smooth muscle cells is directly related to accelerated atherosclerotic process. Cultured cell growth rate is estimated according to *de novo* DNA synthesis assessed (i.e., [³H]Thymidine Incorporation) according to the amount of Tritium-labeled metabolic precursor incorporated into cellular DNA during incubation period.

### Smooth Muscle Cell Growth as an indicator of Cardiovascular Disease Progression

In response to pathological stimuli, smooth muscle cells first migrate from the media layer to the intima layer of the arterial wall, and then proliferate within the intima layer. These events are crucial in the initial development of atherosclerotic plaques. Formation of atherosclerotic lesions in the intima layer occurs in many cardiovascular diseases including hypertension, atherosclerosis, myocardial ischemia, infarction and stroke. (R. Ross, Cellular Mechanisms of Atherosclerosis, Atherosclerosis Review, 103, Vol. 25, pages 195-200). The present compositions are designed to inhibit the invasion and proliferation of smooth muscle cells and thereby to retard progression of atherosclerosis and arteriosclerosis.

The "composition" used in the following experiments refers to a composition containing the following specific ingredients in the specific amounts: lysine is present at 1 gram, proline is present at 750 mg, arginine is present at 500 mg, ascorbic acid is present at 710 mg, magnesium is present at 50 mg, green tea extract is present at 1 gram, N-acetyl-cysteine is present at 200 mg, selenium is present at 30 mcg, copper is present at 2 mg, and manganese is present at 1 mg. Capsules containing the above-mentioned composition was first dissolved in culture media and diluted to appropriate concentrations prior to use. Data represented in **FIG. 1** show *in vitro* experiments on smooth muscle cells. As is shown in **FIG. 1** shows the effect of XR296 on inhibition of smooth muscle cell growth in comparison to EGCG at equivalent concentrations. [³H] thymidine incorporation in human smooth muscle cells is an indicator of de novo DNA synthesis and cell growth. **FIG. 2** shows the effect of XR296 on inhibition of smooth muscle cell growth in comparison to Ascorbic Acid at equivalent concentrations. [³H] thymidine incorporation in human smooth muscle cells is an indicator of de novo DNA synthesis and cell growth. Thymidine incorporation is expressed as a percentage of the value for the control group (100%).

### Growth Rate Assay for Smooth Muscle Cells

Cell cultures of human aortic smooth muscle cells (SMC) were obtained from Clonetics. SMC were cultured in Dulbecco's modified Eagle medium, supplemented with 100 units/ml penicillin, 0.1 mg/ml streptomycin (hereafter DMEM) and 10% FBS (v/v) at 37°C in a humidified atmosphere containing 5% CO₂, and were split 1:3 to 1:5 upon reaching the confluence. SMC at passages 5-8 were used in experiments.
SMC proliferation was assayed by [³H]-Thymidine incorporation into cellular genetic material. Cells were plated in 24-well plates at a density of 10,000 cells per cm² in 0.5 ml of DMEM supplemented with 2% FBS. The attached cells were supplied every 24 hours with fresh growth medium plus additions, as specified in the protocols. A stock solution of XR296 was prepared daily immediately before addition to cell cultures by solving in DMEM to a concentration of 10 mg/ml, vigorously vortexed for 1 minute, and filtered through a 0.2 µm sterile filter. Cell proliferation was measured 3 days later by the addition of 1 µCi/ml [³H]-thymidine to the cell culture for the last 24 hours of the experiment. Cells were washed three times with cold phosphate-buffered saline (PBS), pH 7.2, incubated with 10% trichloracetic acid for 15 minutes at 4°C, washed with cold ethanol, air-dried, solubilized in 0.5 N sodium hydroxide, and then neutralized with hydrochloric acid. Samples were mixed with scintillation fluid and counted using a liquid scintillation counter (model 6500 LS, Beckman Instruments, USA). Cellular DNA-incorporated radioactivity was expressed as d/min per well.

### Expression of Cytokines in Smooth Muscle Cells as in Indicator for Autocrine Inflammatory Response

Rationale: Cytokine expression and its involvement in inflammatory responses are known. It has been recently accepted that vascular and smooth muscle pathology manifested in cardiovascular diseases is one of the inflammatory responses during atherosclerosis and hypertension. Interleukin-6 is one of the key cytokines which trigger the inflammation process. Over-expression of interleukin-6 in smooth muscle cells under pathological stimuli may further amplify the inflammatory lesions. The present compositions are designed to inhibit over-expression of cytokine production in smooth muscle cells (in particular, the interleukin-6). By inhibiting the expression of interleukin-6 in smooth muscle cells, the present compositions are a remedy in treating atherosclerosis, and retarding the effects of low level chronic inflammation and inflammatory responses.
As shown in **FIG. 3****,** while some of the ingredients, such as ascorbic acid, N-Acetyl Cysteine, green tea extract, and amino acids such as proline, lysine and arginine are present in relatively larger proportions, than other ingredients in the composition of the invention XR296, in fact the presence of magnesium, manganese, copper, and selenium provided an added beneficial effect. Thus, the presence of all of these ingredients, including the minerals, provides a synergistic effect not seen by the other ingredients alone or in combination with each other but without magnesium, selenium, manganese, and copper.

### Cytokine Expression Assay

The level of cellular cytokine (IL-6, IL-1 Beta, MCP-1, P-Selectin, and ICAM-1) production is an indicator of stimulation of an inflammatory response. Furthermore, the production of these cytokines stimulates various phases of an inflammatory response. For example, IL-6 and IL-1 Beta are mediators of basic inflammatory response, MCP-1 is a chemo-attractant which attracts monocytes from the blood stream to the artery wall, and P-Selectin and ICAM-1 mediate adhesion of leukocytes to arterial wall cells (endothelium and smooth muscle cells).

Cell culture plastic ware was supplied by Costar and cell media components by GIBCO. Other reagents were from Sigma. Human aortic smooth muscle cells (SMC; Clonetics) have been cultured in DMEM supplemented with penicillin/streptomycin and 10% fetal bovine serum (FBS) at 37°C and 5% CO₂ and used for experiments at passages 5^{th}-8^{th}. For cytokine expression experiments SMC were plated into 24 well plastic plates at 50,000 cells per well and grown to confluence. Cell culture medium was replaced with 0.5 mL serum-free DMEM supplemented with 0.1% bovine serum protein and indicated amounts of nutrient mixture. After 24 hour incubation media were replaced with fresh DMEM/BSA media containing the same amounts of nutrient mixture and a stimulator, 10 ng/mL of tumor necrosis factor alpha (TNFa) or 0.1 mg/mL bacterial lipopolysaccharide (LPS), or no stimulator as control. In 24 hour incubation conditioned media were collected and frozen at -80°C individually for cytokine assay. Cell protein was measured by BCA protein micromethod (Pearce) after cell layer washing with phosphate buffered saline (PBS) and dissolving in 0.1 N NaOH for 2 hour at 37°C. Cell protein per well content was not significantly different from control unsupplemented samples at any used experimental conditions indicating unimpaired cell viability. The level of cytokines in cell conditioned media was assayed with ELISA kits (Quantikine, R&D Systems) accordingly with the manufacturer's protocol. All experiments were performed at least twice in triplicates. Results of a representative experiment are presented as mean cytokine concentrations (+/- SD) in experimental sample.

## Claims

1. Use of a composition comprising lysine, proline, arginine, vitamin C, magnesium, green tea extract, N-acetyl-cysteine, selenium, copper, manganese and one pharmaceutical acceptable component selected from the group consisting of a carrier, a diluent, and an excipient, wherein the composition without the pharmaceutical acceptable component contains 7-9 wt % magnesium, 20-30 wt % ascorbic acid and 11-25 wt % green tea extract for the preparation of a pharmaceutical composition for alleviating or retarding cardiovascular diseases which are **characterized by** excessive smooth muscle cell proliferation.

2. The use of claim 1, wherein said disease is atherosclerosis.

3. The use of claims 1 or 2, wherein the pharmaceutical composition is to be administered orally, intravenously or parenterally.

4. The use of any one of claims 1 to 3, wherein the composition provides the following compounds in the ratio of approximately 25,000 parts of lysine, approximately 15,000 part of proline, approximately 8,000 parts of arginine, approximately 80,000 parts of ascorbic acid, approximately 30,000 parts of magnesium, approximately 50,000 parts of green tea extract, approximately 15,000 parts of N-acetyl-cysteine, approximately 5 parts of selenium, approximately 50 parts of copper, and approximately 200 parts of manganese.

5. The use of any one of claims 1 to 3, wherein the composition provides a dosage of approximately 25 mg of lysine, approximately 15 mg of proline, approximately 8 mg of arginine, approximately 80 mg of ascorbic acid, approximately 30 mg of magnesium, approximately 50 mg of green tea extract, approximately 15 mg of N-acetyl-cysteine, approximately 5 mcg of selenium, approximately 50 mcg of copper, and approximately 200 mcg of manganese.

6. The use of any one of claims 1 to 4, wherein the composition provides a daily dosage of approximately 0.3 mg/kg lysine, 0.2 mg/kg proline, 0.1 mg/kg arginine, 1.1 mg/kg Vitamin C, 0.4 mg/kg magnesium, 0.7 mg/kg green tea extract, and 0.2 mg/kg N-acetyl-cysteine.

7. The use of any one of claims 1 to 5, wherein the composition further comprises one or more of the following substances: Vitamin A, Vitamin D3, Vitamin E, Vitamin B1, Vitamin B2, Niacin, Vitamin B6, Folic Acid, Vitamin B12, Biotin, Pantothenic Acid, Calcium, Phosphorus, Zinc, Chromium, Molybdenum, Pottasium, Citrus Bioflavonoids, Inositol, L-Carnitine, CoEnzyme Q10, Glucosamine, Taurine, and Chondroitin Sulfate.

8. The use of claim 7, wherein the substances are comprised by said composition in the following ratios: 191 IU (International Units) of Vitamin A, 20 IU of Vitamin D3, 10 IU of Vitamin E, 1,500 parts of Vitamin B1, 1,500 parts of Vitamin B2, 10,000 parts of Niacin, 1,500 parts of Vitamin B6, 75 parts of folic acid, 3.3 parts of Vitamin B 12, 10 parts of Biotin, 5,000 parts of Pantothenic Acid, 15,000 parts of Calcium, 2,500 parts of Phosphorus, 2,500 parts of Zinc, 5 parts of Chromium, 0.5 parts of Molybdenum, 5,000 parts of Pottasium, 15,000 parts of Citrus Bioflavonoids, 5,000 parts of Inositol, 5,000 parts of L-Carnitine, 2,500 parts of CoEnzyme Q10, 25,000 parts of-Glucosamine, especially N-Acetyl-D-Glucosamine, 50,000 parts of Taurine, and 15,000 parts of Chondroitin Sulfate.

9. The use of claim 7, wherein the composition comprises one or more of the following substances in the following amounts: approximately 191 IU of Vitamin A, approximately 20 IU of Vitamin D3, approximately 10 IU of Vitamin E, approximately 1.5 mg of Vitamin B1, approximately 1.5 mg of Vitamin B2, approximately 10 mg of Niacin, approximately 1.5 mg of Vitamin B6, approximately 75 mcg of folic acid, approximately 3.3 mcg of Vitamin B12, approximately 10 mcg of Biotin, approximately 5 mg of Pantothenic Acid, approximately 15 mg of Calcium, approximately 2.5 mg of Phosphorus, approximately 2.5 mg of Zinc, approximately 5 mcg of Chromium, approximately 0.5 mcg of Molybdenum, approximately 5 mg of Pottasium, approximately 15 mg of Citrus Bioflavonoids, approximately 5 mg of Inositol, approximately 5 mg of L-Carnitine, approximately 2.5 mg of CoEnzyme Q10, approximately 25 mg of Glucosamine, especially N-Acetyl-D-Glucosamine, approximately 50 mg of Taurine, and/or approximately 15 mg of Chondroitin Sulfate.

10. The use of any one of claims 1 to 9, wherein the composition is in an oral form.

11. The use of claim 10, wherein the oral form is a tablet, a pill or a capsule.

12. The use of any one of claims 1 to 9, wherein the composition is in parenteral form.

## Patentansprüche

1. Verwendung einer Zusammensetzung, die Lysin, Prolin, Arginin, Vitamin C, Magnesium, Extrakt aus Grünem Tee, N-Acetylcystein, Selen, Kupfer, Mangan und eine pharmazeutisch akzeptable Komponente, die aus der Gruppe ausgewählt ist, die aus einem Träger, einem Verdünnungsmittel und einem Hilfsstoff besteht, umfasst, wobei die Zusammensetzung ohne die pharmazeutisch akzeptable Komponente 7-9 Gew.-% Magnesium, 20-30 Gew.-% Ascorbinsäure und 11-25 Gew.-% Extrakt aus Grünem Tee für die Herstellung einer pharmazeutischen Zusammensetzung zur Milderung oder Verzögerung von kardiovaskulären Erkrankungen, die durch eine übermäßige Proliferation von glatten Muskelzellen charakterisiert sind, enthält.

2. Die Verwendung nach Anspruch 1, wobei die Erkrankung Atherosklerose ist.

3. Die Verwendung nach Anspruch 1 oder 2, wobei die pharmazeutische Zusammensetzung oral, intravenös oder parenteral zu verabreichen ist.

4. Die Verwendung nach einem der Ansprüche 1 bis 3, wobei die Zusammensetzung die folgenden Komponenten in einem Verhältnis von cirka 25.000 Teilen Lysin, cirka 15.000 Teilen Prolin, cirka 8.000 Teilen Arginin, cirka 80.000 Teilen Ascorbinsäure, cirka 30.000 Teilen Magnesium, cirka 50.000 Teilen Extrakt aus Grünem Tee, cirka 15.000 Teilen N-Acetylcystein, cirka 5 Teilen Selen, cirka 50 Teilen Kupfer und cirka 200 Teile Mangan bereitstellt.

5. Die Verwendung nach einem der Ansprüche 1 bis 3, wobei die Zusammensetzung eine Dosis von cirka 25 mg Lysin, cirka 15 mg Prolin, cirka 8 mg Arginin, cirka 80 mg Ascorbinsäure, cirka 30 mg Magnesium, cirka 50 mg Extrakt aus Grünem Tee, cirka 15 mg N-Acetylcystein, cirka 5 mcg Selen, cirka 50 mcg Kupfer und cirka 200 mcg Mangan bereitstellt.

6. Die Verwendung nach einem der Ansprüche 1 bis 4, wobei die Zusammensetzung eine tägliche Dosis von cirka 0,3 mg/kg Lysin, 0,2 mg/kg Prolin, 0,1 mg/kg Arginin, 1,1 mg/kg Vitamin C, 0,4 mg/kg Magnesium, 0,7 mg/kg Extrakt aus Grünem Tee und 0,2 mg/kg N-Acetylcystein bereitstellt.

7. Die Verwendung nach einem der Ansprüche 1 bis 5, wobei die Zusammensetzung weiterhin eine oder mehrere der folgenden Substanzen umfasst: Vitamin A, Vitamin D3, Vitamin E, Vitamin B1, Vitamin B2, Niacin, Vitamin B6, Folsäure, Vitamin B12, Biotin, Pantothensäure, Calcium, Phosphor, Zink, Chrom, Molybdän, Kalium, Citrus-Bioflavonoide, Inositol, L-Carnitin, Coenzym Q10, Glucosamin, Taurin und Chondroitinsulfat.

8. Die Verwendung nach Anspruch 7, wobei die Substanzen in der Zusammensetzung in den folgenden Anteilen enthalten sind: 191 IU (Internationale Einheiten) Vitamin A, 20 IU Vitamin D3, 10 IU Vitamin E, 1.500 Teile Vitamin B1, 1.500 Teile Vitamin B2, 10.000 Teile Niacin, 1.500 Teile Vitamin B6, 75 Teile Folsäure, 3,3 Teile Vitamin B12, 10 Teile Biotin, 5.000 Teile Pantothensäure, 15.000 Teile Calcium, 2.500 Teile Phosphor, 2.500 Teile Zink, 5 Teile Chrom, 0,5 Teile Molybdän, 5.000 Teile Kalium, 15.000 Teile Citrus-Bioflavonoide, 5.000 Teile Inositol, 5.000 Teile L-Carnitin, 2.500 Teile Coenzym Q10, 25.000 Teile Glucosamin, insbesondere N-Acetyl-D-Glucosamin, 50.000 Teile Taurin und 15.000 Teile Chondroitinsulfat.

9. Die Verwendung nach Anspruch 7, wobei die Zusammensetzung eine oder mehrere der folgenden Substanzen in den folgenden Mengen umfasst: cirka 191 IU Vitamin A, cirka 20 IU Vitamin D3, cirka 10 IU Vitamin E, cirka 1,5 mg Vitamin B1, cirka 1,5 mg Vitamin B2, cirka 10 mg Niacin, cirka 1,5 mg Vitamin B6, cirka 75 mcg Folsäure, cirka 3,3 mcg Vitamin B12, cirka 10 mcg Biotin, cirka 5 mg Pantothensäure, cirka 15 mg Calcium, cirka 2,5 mg Phosphor, cirka 2,5 mg Zink, cirka 5 mcg Chrom, cirka 0,5 mcg Molybdän, cirka 5 mg Kalium, cirka 15 mg Citrus-Bioflavonoide, cirka 5 mg Inositol, cirka 5 mg L-Carnitin, cirka 2,5 mg Coenzym Q10, cirka 25 mg Glucosamin, insbesondere N-Acetyl-D-Glucosamin, cirka 50 mg Taurin und/oder cirka 15 mg Chondroitinsulfat.

10. Die Verwendung nach einem der Ansprüche 1 bis 9, wobei die Zusammensetzung in einer oralen Form ist.

11. Die Verwendung nach Anspruch 10, wobei die orale Form eine Tablette, ein Dragee oder eine Kapsel ist.

12. Die Verwendung nach einem der Ansprüche 1 bis 9, wobei die Zusammensetzung in parenteraler Form ist.

## Revendications

1. Utilisation d'une composition comprenant de la lysine, de la proline, de l'arginine, de la vitamine C, du magnésium, de l'extrait de thé vert, de la N-acétylcystéine, du sélénium, du cuivre, du manganèse et un composant pharmaceutiquement acceptable choisi parmi le groupe constitué par un véhicule, un diluant et un excipient, où la composition sans le composant pharmaceutiquement acceptable contient 7-9% en poids de magnésium, 20-30% en poids d'acide ascorbique et 11-25% en poids d'extrait de thé vert, pour la préparation d'une composition pharmaceutique destinée à soulager ou retarder les maladies cardiovasculaires qui sont **caractérisées par** une prolifération excessive des cellules des muscles lisses.

2. Utilisation selon la revendication 1, dans laquelle ladite maladie est l'athérosclérose.

3. Utilisation selon les revendications 1 ou 2, dans laquelle la composition pharmaceutique est prévue pour une administration par voie orale, intraveineuse ou parentérale.

4. Utilisation selon l'une quelconque des revendications 1 à 3, dans laquelle la composition fournit les composés suivants selon un rapport d'environ 25 000 parties de lysine, environ 15 000 parties de proline, environ 8 000 parties d'arginine, environ 80 000 parties d'acide ascorbique, environ 30 000 parties de magnésium, environ 50 000 parties d'extrait de thé vert, environ 15 000 parties de N-acétylcystéine, environ 5 parties de sélénium, environ 50 parties de cuivre, et environ 200 parties de manganèse.

5. Utilisation selon l'une quelconque des revendications 1 à 3, dans laquelle la composition fournit un dosage d'environ 25 mg de lysine, environ 15 mg de proline, environ 8 mg d'arginine, environ 80 mg d'acide ascorbique, environ 30 mg de magnésium, environ 50 mg d'extrait de thé vert, environ 15 mg de N-acétylcystéine, environ 5 µg de sélénium, environ 50 µg de cuivre, et environ 200 µg de manganèse.

6. Utilisation selon l'une quelconque des revendications 1 à 4, dans laquelle la composition fournit un dosage journalier d'environ 0,3 mg/kg de lysine, 0,2 mg/kg de proline, 0,1 mg/kg d'arginine, 1,1 mg/kg de vitamine C, 0,4 mg/kg de magnésium, 0,7 mg/kg d'extrait de thé vert, et 0,2 mg/kg de N-acétylcystéine.

7. Utilisation selon l'une quelconque des revendications 1 à 5, dans laquelle la composition comprend en outre une ou plusieurs des substances suivantes : vitamine A, vitamine D3, vitamine E, vitamine B1, vitamine B2, niacine, vitamine B6, acide folique, vitamine B12, biotine, acide pantothénique, calcium, phosphore, zinc, chrome, molybdène, potassium, bioflavonoïdes de citrus, inositol, L-carnitine, coenzyme Q10, glucosamine, taurine et sulfate de chondroïtine.

8. Utilisation selon la revendication 7, dans laquelle les substances sont comprises par ladite composition selon les rapports suivants : 191 UI (Unités Internationales) de vitamine A, 20 UI de vitamine D3, 10 UI de vitamine E, 1500 parties de vitamine B1, 1500 parties de vitamine B2, 10 000 parties de niacine, 1500 parties de vitamine B6, 75 parties d'acide folique, 3,3 parties de vitamine B12, 10 parties de biotine, 5000 parties d'acide pantothénique, 15 000 parties de calcium, 2500 parties de phosphore, 2500 parties de zinc, 5 parties de chrome, 0,5 partie de molybdène, 5000 parties de potassium, 15 000 parties de bioflavonoïdes de citrus, 5000 parties d'inositol, 5000 parties de L-carnitine, 2500 parties de coenzyme Q10, 25 000 parties de glucosamine, notamment de N-acétyl-D-glucosamine, 50 000 parties de taurine et 15 000 parties de sulfate de chondroïtine.

9. Utilisation selon la revendication 7, dans laquelle la composition comprend une ou plusieurs des substances suivantes selon les quantités suivantes : environ 191 UI de vitamine A, environ 20 UI de vitamine D3, environ 10 UI de vitamine E, environ 1,5 mg de vitamine B1, environ 1,5 mg de vitamine B2, environ 10 mg de niacine, environ 1,5 mg de vitamine B6, environ 75 µg d'acide folique, environ 3,3 µg de vitamine B12, environ 10 µg de biotine, environ 5 mg d'acide pantothénique, environ 15 mg de calcium, environ 2,5 mg de phosphore, environ 2,5 mg de zinc, environ 5 µg de chrome, environ 0,5 µg de molybdène, environ 5 mg de potassium, environ 15 mg de bioflavonoïdes de citrus, environ 5 mg d'inositol, environ 5 mg de L-carnitine, environ 2,5 mg de coenzyme Q10, environ 25 mg de glucosamine, notamment de N-acétyl-D-glucosamine, environ 50 mg de taurine et/ou environ 15 mg de sulfate de chondroïtine.

10. Utilisation selon l'une quelconque des revendications 1 à 9, dans laquelle la composition est sous forme orale.

11. Utilisation selon la revendication 10, dans laquelle la forme orale est un comprimé, une pilule ou une capsule.

12. Utilisation selon l'une quelconque des revendications 1 à 9, dans laquelle la composition est sous forme parentérale.
